(19) Europäisches Patentamt / European Patent Office / Office européen des brevets

(11) **EP 4 355 291 B1**

(12) **EUROPÄISCHE PATENTSCHRIFT**

(45) Veröffentlichungstag und Bekanntmachung des Hinweises auf die Patenterteilung:
**21.05.2025 Patentblatt 2025/21**

(21) Anmeldenummer: **22733580.9**

(22) Anmeldetag: **09.06.2022**

(51) Internationale Patentklassifikation (IPC):
*A61K 8/37* (2006.01)    *A61Q 19/00* (2006.01)
*A61K 8/85* (2006.01)

(52) Gemeinsame Patentklassifikation (CPC):
**A61K 8/37; A61K 8/85; A61Q 19/00;** A61K 2800/87

(86) Internationale Anmeldenummer:
**PCT/EP2022/065659**

(87) Internationale Veröffentlichungsnummer:
**WO 2022/263276 (22.12.2022 Gazette 2022/51)**

(54) **KOSMETIKUM AUS EINEM PACKMITTEL UND EINER ZUBEREITUNG ENTHALTEND ETHYLHEXYLOXYSTEARATESTER**

COSMETIC PRODUCT CONSISTING OF PACKAGING AND A PREPARATION CONTAINING ETHYLHEXYL OXYSTEARATE ESTER

PRODUIT COSMÉTIQUE CONSTITUÉ D'UN MATÉRIAU D'EMBALLAGE ET D'UNE PRÉPARATION CONTENANT DES ESTERS DE STÉARATE D'ÉTHYLHEXYLOXY

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.06.2021 DE 102021206049**

(43) Veröffentlichungstag der Anmeldung:
**24.04.2024 Patentblatt 2024/17**

(73) Patentinhaber: **Beiersdorf AG**
**22529 Hamburg (DE)**

(72) Erfinder:
• **MÜLLER, Claudia**
**25499 Tangstedt (DE)**
• **NEBEN, Jette Mareike**
**22527 Hamburg (DE)**
• **DIEKERT, Sabrina**
**22761 Hamburg (DE)**
• **LEHMBECK, Frank**
**22848 Norderstedt (DE)**

(74) Vertreter: **Beiersdorf AG**
**Patentabteilung**
**Beiersdorfstraße 1 - 9**
**22529 Hamburg (DE)**

(56) Entgegenhaltungen:
CN-A- 102 762 661    FR-A1- 2 290 414
GB-A- 2 077 620

**Beschreibung**

Die vorliegende Erfindung betrifft ein Kosmetikum aus einer kosmetischen Zubereitung enthaltend Ethylhexyloxystearatester sowie einem Packmittel aus HDPE, LDPE, PP oder PET.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren immer das Ziel der Menschen gewesen. Einen wesentlichen Anteil an einem schönen und attraktiven Äußeren hat dabei der Zustand und das Aussehen der Haut.

Damit die Haut ihre biologischen Funktionen im vollen Umfang erfüllen kann, bedarf sie der regelmäßigen Reinigung und Pflege. Die Reinigung der Haut dient dabei der Entfernung von Schmutz, Schweiß und Resten abgestorbener Hautpartikel, die einen idealen Nährboden für Krankheitserreger und Parasiten aller Art bilden. Hautpflegeprodukte dienen meist der Befeuchtung und Rückfettung der Haut. Häufig sind ihnen Wirkstoffe zugesetzt, welche die Haut regenerieren und beispielsweise ihre vorzeitige Alterung (z.B. das Entstehen von Fältchen, Falten) verhindern und vermindern sollen.

Hautpflegeprodukte werden in der Regel in Packmitteln in Form von Flaschen, Tuben oder Tiegeln aufbewahrt. Diese bestehen überwiegend aus Glas (Glas-Tiegel) oder Kunststoffen wie Polyethylen hoher Dichte (HDPE), Polyethylen geringer Dichte (LDPE), Polypropylen (PP) oder Polyethylenterephthalat (PET).

Nachteilig an derartigen an sich sehr günstigen und außerordentlich hygienischen Packmitteln ist der Umstand, dass diese Packmittel nicht besonders nachhaltig/umweltfreundlich sind, da sie aus Erdölprodukten und/oder Erdgas hergestellt werden und bei ihrer thermischen Verwertung (Verbrennung in der Müllverbrennungsanlage) letztlich Kohlendioxid aus fossilen Quellen freigesetzt wird.

Um die natürlichen Ressourcen zu schonen, wird daher versucht, diese Packmittel möglichst dünnwandig herzustellen und nach der Verwendung der Zubereitung zu recyclen.

Diese dünnwandigen Packmittel haben jedoch den Nachteil, dass sie, wenn die in ihr abgefüllte kosmetische Zubereitung ein oder mehrere Ölkomponenten enthält, nicht besonders lagerbeständig sind, sondern vielmehr bei Kontakt mit dem Öl aufquellen, da das Öl vom Kunststoff "aufgesaugt" und absorbiert wird. Darüber hinaus können die Produkte Ausölen. Ferner führt das Öl im Kunststoff dazu, dass auf dem Packmittel aufgedruckte Beschriftungen verwischen und sich ablösen können. Außerdem erschwert das vom Packmittel aufgenommene Öl das Recycling des Kunststoffes, weil das Öl aufwändig vom Polymer getrennt werden muss, bevor aus dem Polymer-Kunststoff wieder ein neues Packmittel hergestellt werden kann. Aus diesen Gründen können in herkömmlichen, dünnwandigen Packmitteln entweder nur ölfreie Zubereitungen (z.B. Zahnpasta) abgefüllt werden oder das Packmittel muss auf der dem Kosmetikum zugewandten Innenseite aufwändig beschichtet bzw. versiegelt werden. Aus dem Stand der Technik bekannt sind beispielsweise Schutzbarrieren aus Ethylenvinylalkohol, die relativ teuer und ebenfalls weniger gut zu recyclen sind als reine Polyolefin-Packmittel.

CN102762661 A offenbart eine Verpackungskomponente, die ein bestimmtes Harzmaterial umfasst, und ein Verfahren zum Verhindern der Adsorption lipophiler Substanzen aus wässrigen Zusammensetzungen, das dieses verwendet.

Weiter verstärkt wird das Problem der Aufnahme von Ölen aus der Zubereitung durch das Packmittel durch den generellen Trend, bestimmte Ölkomponenten aus technischer Herstellung, beispielsweise wegen ihrer schlechten Abbaubarkeit oder hoher Emissionswerte bei der Herstellung, aus kosmetischen Zubereitungen zu verbannen. Insbesondere Dimethicone war/ist bei Produktentwicklern an sich wegen seiner geringen Neigung zur Migration in Kunststoff-Verpackungen, besonders beliebt. Andererseits wird aus unterschiedlichen Gründen gefordert, kosmetische Zubereitungen ohne Dimethicon zu formulieren.

Es war daher die Aufgabe der vorliegenden Erfindung, die Nachteile des Standes der Technik zu reduzieren und im Idealfalle zu beseitigen und ein Kosmetikum aus einer ölhaltigen kosmetischen Zubereitung und einer Kunststoffverpackung zu entwickeln, dessen Packmittel einfacher zu recyclen und lagerstabil ist, d.h. bei dem die Ölabsorption der Kunststoffverpackung deutlich reduziert ist, ohne das auf dem Kunststoff eine Schutzschicht (z.B. aus Ethylenvinylalkohol) aufgetragen werden muss.

Überraschend gelöst wird die Aufgabe durch ein Kosmetikum aus

a) einem Packmittel aus Polyethylen hoher Dichte (HDPE), Polyethylen geringer Dichte (LDPE), Polypropylen (PP) oder Polyethylenterephthalat (PET), sowie
b) einer kosmetischen Zubereitung enthaltend Ethylhexyloxystearatester.

Zwar kennt der Fachmann Kosmetika aus Packmitteln mit HDPE, PP oder PET, in denen kosmetische Zubereitungen mit Esterölen wie beispielsweise C12-15 Alkylbenzoat, oder Etheröle wie beispielsweise Dicaprylylether enthalten sind, doch neigen diese Ölkomponenten des Standes der Technik dazu, relativ stark in diese Verpackungsmaterialien zu migrieren.

Daher weisen derartige Produkte üblicherweise eine Schutzschicht aus z.B. Ethylemvinylalkohol auf der Innenseite auf, die üblicherweise nicht auf dem Packmittel deklariert wird bzw. deklariert werden muss.

Es ist erfindungsgemäß bevorzugt, wenn das Packmittel a) aus Polyethylen hoher Dichte (HDPE) gebildet wird.

Ferner ist es erfindungsgemäß vorteilhaft, wenn das Packmittel a) eine Wandstärke (Dicke) von 1 bis 8 mm aufweist.

Darüber hinaus sind die erfindungsgemäß vorteilhaften Ausführungsformen der vorliegenden Erfindung dadurch gekennzeichnet, dass das Packmittel auf der Innenseite, welche der Zubereitung zugewandt ist, keine Schutzschicht (z.B. aus Ethylenvinylalkohol) aufweist.

Es ist erfindungsgemäß bevorzugt, wenn als Ethylhexyloxystearatester Ethylhexylacetoxystearat (INCI: Ethylhexyl Acetoxystearate) und/oder Acetylethylhexylpolyhydroxystearat (INCI: Acetyl Ethylhexyl Polyhydroxystearate) eingesetzt wird.

Erfindungsgemäß vorteilhafte Ausführungsformen der vorliegenden Erfindung werden auch dadurch erhalten, dass die Zubereitung frei ist von Dimethicon (INCI: Dimethicone).

Darüber hinaus ist es erfindungsgemäß von Vorteil, wenn die Zubereitung frei ist von Silikonöl, Mineralöl, Paraffinwachs, mikrokristallinem Wachs, Schellackwachs und Polyethylenwachsen.

Erfindungsgemäß vorteilhaft ist es darüber hinaus, wenn die Zubereitung frei ist von Polyacrylaten, quervernetzten Acrylat/C10-C30 Alkylacrylat Polymeren und Vinylpyrrolidon/ Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethylisothiazolinon und DMDM-Hydantoin, Polyethylenglycolethern oder Polyethylenglycolestern

Vorteilhaft im Sinne der vorliegenden Erfindung ist es darüber hinaus, wenn die kosmetische Zubereitung in Form einer Emulsion vorliegt. Dabei ist es erfindungsgemäß bevorzugt, wenn die kosmetische Zubereitung in Form einer Öl-in-Wasser-Emulsion (O/W-Emulsion) vorliegt.

Es ist erfindungsgemäß von Vorteil, wenn die kosmetische Zubereitung Ethylhexyloxystearatester in einer Konzentration von 0,1 bis 10 Gewichts-%, bezogen auf das Gesamtgewicht der Zubereitung, enthält. Dabei ist eine Einsatzkonzentration von 0,1 bis 5 Gewichts-%, bezogen auf das Gesamtgewicht der Zubereitung, erfindungsgemäß bevorzugt. Es beziehen sich diese Einsatzkonzentrationen dabei auf die Gesamtmenge an eingesetzten Ethylhexyloxystearatestern. Wird nur ein Ethylhexyloxystearatester (z.B. Ethylhexylacetoxystearat (INCI: Ethylhexyl Acetoxystearate) oder Acetylethylhexylpolyhydroxystearat (INCI: Acetyl Ethylhexyl Polyhydroxystearate) eingesetzt, ist dies die vorteilhafte bzw. bevorzugte Einsatzkonzentration für diese eine Verbindung.

Erfindungsgemäß vorteilhaft ist es darüber hinaus, wenn die Gesamtmenge an Ethylhexyloxystearatestern in der Ölphase der Zubereitung mindestens 3 Gewichts-%, bezogen auf das Gesamtgewicht der Ölphase, beträgt.

Erfindungsgemäß vorteilhafte Ausführungsformen sind auch dadurch gekennzeichnet, dass die Zubereitung ein oder mehrere Öle gewählt aus der Gruppe der Verbindungen Octyldodecanol, Capryl/Caprinsäure Trigyceride (INCI: Caprylic/Capric Triglyceride), Kokosglyceride (INCI: Cocoglycerides), Tridecylstearat (INCI: Tridecyl Stearate), Tridecyltrimelliat (INCI: Tridecyl Trimelliate) enthält.

Ferner ist es erfindungsgemäß von Vorteil, wenn die Zubereitung ein oder mehrere Öle gewählt aus der Gruppe der Verbindungen enthaltend Polycitronellol (INCI: Polycitronellol), Polycitronellolacetat (INCI: Polycitronellol Acetate), Triolein, Propylenglycoldiheptanoat (INCI: Propylene Glycol Diheptanoate), Propylenglycoldicaprat (INCI: Propylene Glycol Dicaprate), Propylenglycoldicaprylat/Dicaprat (INCI: Propylene Glycol Dicaprylate/Dicaprate) und Propylenglycoldibenzoat (INCI: Propylene Glycol Dibenzoate) enthält.

Außerdem ist es erfindungsgemäß vorteilhaft, wenn die Zubereitung ein oder mehrere Öle gewählt aus der Gruppe der Verbindungen Mandelöl (INCI: Prunus Amygdalus Dulcis Oil) und/oder Sonnenblumenöl (INCI: Helianthus Annuus Seed Oil), hydriertes Rizinusöl (INCI: Hydrogenated Castor Oil) enthält.

Darüber hinaus ist es erfindungsgemäß von Vorteil, wenn die Zubereitung Tocopherol enthält.

Ansonsten kann die erfindungsgemäße Zubereitung die für Kosmetika üblichen Inhaltsstoffe enthalten und entsprechend zusammengesetzt sein.

## Vergleichsversuche

Es wurde die Penetration der folgenden kosmetischen Öle in das Verpackungsmaterial HDPE untersucht. Dabei wurde als HDPE das Produkt "Hostalen ACP 5831D" der Firma Lyondellbasell eingesetzt.

## Testdurchführung

1. Drei Stanzlinge aus dem Verpackungsmaterial mit einem Durchmesser von 5 mm und einer Höhe von 1 mm für jeden Rohstoff/ jede kosmetische Zubereitung verwenden.

2. Das Ausgangsgewicht jedes Stanzlings auf der Analysenwaage bestimmen. (Ausgangsgewicht = m0)

3. Die Stanzlinge in ein verschließbares Gefäß geben und den Rohstoff / die kosmetische Zubereitung zugeben. Die Stanzlinge müssen von dem Rohstoff / der kosmetischen Zubereitung komplett umschlossen sein.

4. Das geschlossene Gefäß bei 40 ± 1°C im Brutschrank lagern.

5. Mechanische Reinigung der Stanzlinge mit Zellstoff nach 28 Tagen.

5. Die Stanzlinge nach 28 Tagen erneut wiegen.

| Gewicht des Musters (Stanzling) | Bestimmung |
|---|---|
| m0 | Ausgangsgewicht |
| m1 | 28 Tage |

**Hinweise**

Die Stanzlinge nicht mit dem Fingern berühren, zur Vermeidung von Kontamination immer eine Pinzette verwenden.

**Auswertung**

Rohstoffe oder Bestandteile der kosmetischen Zubereitung sind in das Verpackungsmaterial migriert, wenn das Gewicht des Stanzlings m1 größer als das Gewicht von m0 ist.

$$\text{Gewichtserhöhung (\%)} = (m1 - m0) / m0$$

Mit der Formel (m1 - m0)/m0 wird die Gewichtserhöhung (%) des Rohstoffes oder der kosmetischen Zubereitung bestimmt, die in das Verpackungsmaterial migriert ist.

Es werden zusätzlich Mittelwert [%] und Standardabweichung [%] der Dreifachbestimmung errechnet.

**Ergebnisse**

| INCI | Gewichtserhöhung in % nach 28d | |
|---|---|---|
| | Mittelwert [%] | Standardabweichung [%] |
| Ethylhexylacetoxystearat (INCI: Ethylhexyl Acetoxystearate) | 2,06 | 0,08 |
| Acetylethylhexylpolyhydroxystearat (INCI: Acetyl Ethylhexyl Polyhydroxystearate) | 0,98 | 0,01 |
| Paraffinum Liquidum | 5,24 | 0,05 |
| C12-15 Alkylbenzoat | 4,85 | 0,04 |
| Dicaprylylether | 5,52 | 0,04 |

**Beispiele**

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| INCI | O/W Lotion | W/O Lotion |
|---|---|---|
| | Gew. % | Gew. % |
| Acetyl Ethylhexyl Polyhydroxystearate | 3,5 | 5 |
| Ethylhexylglycerin | 0,2 | 0 |
| Butyrospermum Parkii Butter | 1,2 | 1 |
| Cetyl Palmitate | 0 | 0,6 |
| Isopropyl Palmitate | 0 | 5 |
| Cocoglycerides | 0 | 5 |
| Coco-Caprylate/Caprate | 0 | 5 |
| Sodium Cetearyl Sulfate | 0,1 | 0 |
| Glyceryl Stearate SE | 2,2 | 0 |
| Diisostearoyl Polyglyceryl-3 Dimer Dilinoleate | 0 | 1,4 |
| Polyglyceryl-4 Diisostearate/Polyhydroxystearate/Sebacate | 0 | 0,8 |
| Glycerin | 7 | 7,5 |
| Citric Acid | 0 | 0,09 |
| Sodium Citrate + Aqua | 0 | 0,175 |
| Phenoxyethanol | 1 | 0 |
| Potassium Sorbate | 0 | 0,13 |
| Cetearyl Alcohol | 4,5 | 0 |
| Xanthan Gum | 0,1 | 0 |
| Hydroxypropyl Starch Phosphate + Aqua | 0,5 | 0 |
| Magnesium Sulfate | 0 | 0,7 |
| Aqua | ad 100 | ad 100 |

Diese Zubereitungen wurden in ein flaschenförmiges Vorratsgefäß aus HDPE (Hostalen ACP 5831 D, der Firma Lyondellbasell) abgefüllt und aufbewahrt.

**Weitere Beispielrezepturen:**

**Beispiel 1: O/W Lotion**

| INCI | Gew. % |
|---|---|
| Ethylhexyl Acetoxystearate | 3,5 |
| Ethylhexylglycerin | 0,2 |
| Sodium Cetearyl Sulfate | 0,1 |
| Glyceryl Stearate SE | 2 |
| Glycerin | 5 |
| Benzyl Alcohol | 0,2 |
| Cetearyl Alcohol | 5 |
| Helianthus Annuus Seed Oil | 0,1 |
| Xanthan Gum | 0,1 |

(fortgesetzt)

| INCI | Gew. % |
|---|---|
| Alcohol denat. | 5 |
| Hydroxypropyl Starch Phosphate + Aqua | 1,3 |
| Citric Acid (pH: 6,5) | qs |
| Aqua | ad 100 |

**Beispiel 2: W/O Lotion**

| INCI | Gew. % |
|---|---|
| Ethylhexyl Acetoxystearate | 3 |
| Butyrospermum Parkii Butter | 1 |
| Cetyl Palmitate | 0,6 |
| Isopropyl Palmitate | 8 |
| Vegetable Oil | 5 |
| Coco-Caprylate/Caprate | 5 |
| Diisostearoyl Polyglyceryl-3 Dimer Dilinoleate | 1,4 |
| Polyglyceryl-4 Diisostearate/Polyhydroxystearate/Sebacate | 0,8 |
| Glycerin | 5 |
| Citric Acid | 0,09 |
| Sodium Citrate + Aqua | 0,175 |
| Potassium Sorbate | 0,13 |
| Prunus Amygdalus Dulcis Oil | 0,5 |
| Magnesium Sulfate | 0,7 |
| Aqua | ad 100 |

**Beispiel 3: O/W Creme**

| INCI | Gew. % |
|---|---|
| Decylene Glycol | 0,1 |
| Ethylhexyl Polyhydroxystearate | 3,5 |
| Levulinic Acid + Aqua + Glycerin + Sodium Levulinate | 0,5 |
| Tocopherol | 0,01 |
| Caprylic/Capric Triglyceride | 3,5 |
| Simmondsia Chinensis Seed Oil | 2 |
| Butyrospermum Parkii Butter | 1,5 |
| Prunus Amygdalus Dulcis Oil | 2 |
| Sodium Stearoyl Glutamate | 0,25 |
| Glyceryl Stearate | 2 |

(fortgesetzt)

| INCI | Gew. % |
|---|---|
| Glyceryl Caprylate | 0,2 |
| Parfum | 0,2 |
| Glycerin | 7,5 |
| Cetearyl Alcohol | 3,5 |
| Alcohol | 6 |
| Aqua | Ad 100 |

**Beispiel 4: O/W Creme**

| INCI | Gew.% |
|---|---|
| Ethylhexyl Acetoxystearate | 3,0 |
| Levulinic Acid + Aqua + Glycerin + Sodium Levulinate | 0,5 |
| Ethylhexylglycerin | 0,2 |
| Aloe Barbadensis Leaf Juice Powder | 0,4 |
| Coco-Caprylate/Caprate | 1 |
| Simmondsia Chinensis Seed Oil | 3,5 |
| Butyrospermum Parkii Butter | 0,5 |
| Prunus Amygdalus Dulcis Oil | 3 |
| Dicaprylyl Ether | 1 |
| Sodium Stearoyl Glutamate | 0,3 |
| Glyceryl Stearate | 2 |
| Distarch Phosphate | 1 |
| Glyceryl Caprylate | 0,2 |
| Parfum | 0,2 |
| Glycerin | 7,0 |
| Cetearyl Alcohol | 4,5 |
| Xanthan Gum | 0,3 |
| Alcohol | 4 |
| Aqua | Ad 100 |

**Patentansprüche**

1. Kosmetikum aus

   a) einem Packmittel aus Polyethylen hoher Dichte (HDPE), Polyethylen geringer Dichte (LDPE), Polypropylen (PP) oder Polyethylenterephthalat (PET), sowie
   b) einer kosmetischen Zubereitung enthaltend Ethylhexyloxystearatester.

2. Kosmetikum nach Anspruch 1, **dadurch gekennzeichnet, dass** das Packmittel a) aus Polyethylen hoher Dichte (HDPE) gebildet wird.

3. Kosmetikum nach einem der vorhergehenden Ansprüche, **dadurch gekennzeichnet, dass** das Packmittel a) eine Wandstärke (Dicke) von 1 bis 8 mm aufweist.

4. Kosmetikum nach einem der vorhergehenden Ansprüche, **dadurch gekennzeichnet, dass** das Packmittel auf der Innenseite, welche der Zubereitung zugewandt ist, keine Schutzschicht (z.B. aus Ethylenvinylalkohol) aufweist.

5. Kosmetikum nach einem der vorhergehenden Ansprüche, **dadurch gekennzeichnet, dass** als Ethylhexyloxystearatester Ethylhexylacetoxystearat (INCI: Ethylhexyl Acetoxystearate) und/oder Acetylethylhexylpolyhydroxystearat (INCI: Acetyl Ethylhexyl Polyhydroxystearate) eingesetzt wird.

6. Kosmetikum nach einem der vorhergehenden Ansprüche, **dadurch gekennzeichnet, dass** die Zubereitung frei ist von Dimethicon (INCI: Dimethicone).

7. Kosmetikum nach einem der vorhergehenden Ansprüche, **dadurch gekennzeichnet, dass** die Zubereitung frei ist von Silikonöl, Mineralöl, Paraffinwachs, mikrokristallinem Wachs, Schellackwachs und Polyethylenwachsen.

8. Kosmetikum nach einem der vorhergehenden Ansprüche, **dadurch gekennzeichnet, dass** die Zubereitung frei ist von Polyacrylaten, quervernetzten Acrylat/C10-C30 Alkylacrylat Polymeren und Vinylpyrrolidon/Hexadecen-Copolymeren, sowie frei von 3-(4-Methylbenzyliden)-campher, 2-Hydroxy-4-methoxybenzophenon (INCI: Oxybenzon), 4-Methoxyzimtsäure-2-ethylhexylester (INCI Octylmethoxycinnamate), Ethylhexyl-2-cyano-3,3-diphenylacrylat (INCI: Octocrylen), Parabenen (insbesondere Methyl-, Propyl- und Butylparaben), Methylisothiazolinon, Chlormethyl-isothiazolinon und DMDM-Hydantoin, Polyethylenglycolethern oder Polyethylenglycolestern

9. Kosmetikum nach einem der vorhergehenden Ansprüche, **dadurch gekennzeichnet, dass** die kosmetische Zubereitung in Form einer Emulsion vorliegt.

10. Kosmetikum nach einem der vorhergehenden Ansprüche, **dadurch gekennzeichnet, dass** die kosmetische Zubereitung in Form einer Öl-in-Wasser-Emulsion (O/W-Emulsion) vorliegt.

11. Kosmetikum nach einem der vorhergehenden Ansprüche, **dadurch gekennzeichnet, dass** die kosmetische Zubereitung Ethylhexyloxystearatester in einer Konzentration von 0,1 bis 10 Gewichts-%, bezogen auf das Gesamtgewicht der Zubereitung, enthält.

12. Kosmetikum nach einem der vorhergehenden Ansprüche, **dadurch gekennzeichnet, dass** die Gesamtmenge an Ethylhexyloxystearatester in der Ölphase der Zubereitung mindestens 3 Gewichts-%, bezogen auf das Gesamtgewicht der Ölphase, beträgt.

13. Kosmetikum nach einem der vorhergehenden Ansprüche, **dadurch gekennzeichnet, dass** die Zubereitung ein oder mehrere Öle gewählt aus der Gruppe der Verbindungen Octyldodecanol, Capryl/Caprinsäure Trigyceride (INCI: Caprylic/Capric Triglyceride), Kokosglyceride (INCI: Cocoglycerides), Tridecylstearat (INCI: Tridecyl Stearate), Tridecyltrimelliat (INCI: Tridecyl Trimelliate) enthält.

14. Kosmetikum nach einem der vorhergehenden Ansprüche, **dadurch gekennzeichnet, dass** die Zubereitung ein oder mehrere Öle gewählt aus der Gruppe der Verbindungen enthaltend Polycitronellol (INCI: Polycitronellol), Polycitronellolacetat (INCI: Polycitronellol Acetate), Triolein, Propylenglycoldiheptanoat (INCI: Propylene Glycol Diheptanoate), Propylenglycoldicaprat (INCI: Propylene Glycol Dicaprate), Propylenglycoldicaprylat/Dicaprat (INCI: Propylene Glycol Dicaprylate/Dicaprate) und Propylenglycoldibenzoat (INCI: Propylene Glycol Dibenzoate) enthält.

15. Kosmetikum nach einem der vorhergehenden Ansprüche, **dadurch gekennzeichnet, dass** die Zubereitung Tocopherol enthält.

16. Kosmetikum nach einem der vorhergehenden Ansprüche, **dadurch gekennzeichnet, dass** die Zubereitung ein oder mehrere Öle gewählt aus der Gruppe der Verbindungen Mandelöl (INCI: Prunus Amygdalus Dulcis Oil) und/oder Sonnenblumenöl (INCI: Helianthus Annuus Seed Oil), hydriertes Rizinusöl (INCI: Hydrogenated Castor Oil) enthält.

**Claims**

1. Cosmetic product consisting of

   a) a packaging made of high-density polyethylene (HDPE), low-density polyethylene (LDPE), polypropylene (PP) or polyethylene terephthalate (PET), and
   b) a cosmetic preparation comprising ethylhexyl oxystearate esters.

2. Cosmetic product according to Claim 1, **characterized in that** the packaging a) is formed from high-density polyethylene (HDPE).

3. Cosmetic product according to either of the preceding claims, **characterized in that** the packaging a) has a wall thickness of 1 to 8 mm.

4. Cosmetic product according to any of the preceding claims, **characterized in that** the packaging does not have any protective layer (for example made of ethylene vinyl alcohol) on the inner side facing the preparation.

5. Cosmetic product according to any of the preceding claims, **characterized in that** ethylhexyl acetoxystearate (INCI: Ethylhexyl Acetoxystearate) and/or acetyl ethylhexyl polyhydroxystearate (INCI: Acetyl Ethylhexyl Polyhydroxystearate) is used as ethylhexyl oxystearate ester.

6. Cosmetic product according to any of the preceding claims, **characterized in that** the preparation is free of dimethicone (INCI: Dimethicone).

7. Cosmetic product according to any of the preceding claims, **characterized in that** the preparation is free of silicone oil, mineral oil, paraffin wax, microcrystalline wax, shellac wax and polyethylene waxes.

8. Cosmetic product according to any of the preceding claims, **characterized in that** the preparation is free of polyacrylates, crosslinked acrylate/C10-C30 alkyl acrylate polymers and vinylpyrrolidone/hexadecene copolymers, and free of 3-(4-methylbenzylidene)camphor, 2-hydroxy-4-methoxybenzophenone (INCI: Oxybenzone), 2-ethylhexyl 4-methoxycinnamate (INCI: Octyl Methoxycinnamate), ethylhexyl 2-cyano-3,3-diphenylacrylate (INCI: Octocrylene), parabens (particularly methyl, propyl and butyl paraben), methylisothiazolinone, chloromethylisothiazolinone and DMDM hydantoin, polyethylene glycol ethers or polyethylene glycol esters.

9. Cosmetic product according to any of the preceding claims, **characterized in that** the cosmetic preparation is in the form of an emulsion.

10. Cosmetic product according to any of the preceding claims, **characterized in that** the cosmetic preparation is in the form of an oil-in-water emulsion (O/W emulsion).

11. Cosmetic product according to any of the preceding claims, **characterized in that** the cosmetic preparation comprises ethylhexyl oxystearate esters at a concentration of 0.1% to 10% by weight, based on the total weight of the preparation.

12. Cosmetic product according to any of the preceding claims, **characterized in that** the total amount of ethylhexyl oxystearate esters in the oil phase of the preparation is at least 3% by weight, based on the total weight of the oil phase.

13. Cosmetic product according to any of the preceding claims, **characterized in that** the preparation comprises one or more oils selected from the following group of compounds: octyldodecanol, caprylic/capric triglyceride (INCI: Caprylic/Capric Triglyceride), cocoglycerides (INCI: Cocoglycerides), tridecyl stearate (INCI: Tridecyl Stearate), tridecyl trimellitate (INCI: Tridecyl Trimellitate).

14. Cosmetic product according to any of the preceding claims, **characterized in that** the preparation comprises one or more oils selected from the group of compounds comprising: polycitronellol (INCI: Polycitronellol), polycitronellol acetate (INCI: Polycitronellol Acetate), triolein, propylene glycol diheptanoate (INCI: Propylene Glycol Diheptanoate), propylene glycol dicaprate (INCI: Propylene Glycol Dicaprate), propylene glycol dicaprylate/dicaprate (INCI: Propylene Glycol Dicaprylate/Dicaprate) and propylene glycol dibenzoate (INCI: Propylene Glycol Dibenzoate).

**15.** Cosmetic product according to any of the preceding claims, **characterized in that** the preparation comprises tocopherol.

**16.** Cosmetic product according to any of the preceding claims, **characterized in that** the preparation comprises one or more oils selected from the following group of compounds: almond oil (INCI: Prunus Amygdalus Dulcis Oil) and/or sunflower oil (INCI: Helianthus Annuus Seed Oil), hydrogenated castor oil (INCI: Hydrogenated Castor Oil).

**Revendications**

**1.** Cosmétique constitué par

a) un matériau d'emballage en polyéthylène à haute densité (HDPE), en polyéthylène basse densité (LDPE), en polypropylène (PP) ou en poly(téréphtalate d'éthylène) (PET), ainsi que par
b) une préparation cosmétique contenant un ester de type oxystéarate d'éthylhexyle.

**2.** Cosmétique selon la revendication 1, **caractérisé en ce que** le matériau d'emballage a) est formé à partir de polyéthylène à haute densité (HDPE).

**3.** Cosmétique selon l'une des revendications précédentes, **caractérisé en ce que** le matériau d'emballage a) présente une épaisseur de paroi de 1 à 8 mm.

**4.** Cosmétique selon l'une des revendications précédentes, **caractérisé en ce que** le matériau d'emballage ne présente pas de couche de protection (par exemple en éthylène-alcool vinylique) sur la face interne qui est orientée vers la préparation.

**5.** Cosmétique selon l'une des revendications précédentes, **caractérisé en ce que** de l'acétoxystéarate d'éthylhexyle (INCI : Ethylhexyl Acetoxystearate) et/ou du polyhydroxystéarate d'acétyléthylhexyle (INCI : Acetyl Ethylhexyl Polyhydroxystearate) est/sont utilisé(s) en tant qu'ester de type oxystéarate d'éthylhexyle.

**6.** Cosmétique selon l'une des revendications précédentes, **caractérisé en ce que** la préparation est exempte de diméthicone (INCI : Dimethicone).

**7.** Cosmétique selon l'une des revendications précédentes, **caractérisé en ce que** la préparation est exempte d'huile de silicone, d'huile minérale, de cire de paraffine, de cire microcristalline, de cire de gomme laque et de cires de polyéthylène.

**8.** Cosmétique selon l'une des revendications précédentes, **caractérisé en ce que** la préparation est exempte de polyacrylates, de polymères réticulés d'acrylate/acrylate de C10-C30-alkyle et de copolymères de vinylpyrrolidone/hexadécène, ainsi qu'exempte de 3-(4-méthylbenzylidène)-camphre, de 2-hydroxy-4-méthoxybenzophénone (INCI : Oxybenzon), d'ester 2-éthylhexylique de l'acide 4-méthoxycinnamique (INCI : Octylmethoxycinnamate), de 2-cyano-3,3-diphénylacrylate d'éthylhexyle (INCI : Octocrylen), de parabènes (en particulier de méthylparabène, de propylparabène et de butylparabène), de méthylisothiazolinone, de chlorométhyl-isothiazolinone et de DMDM-hydantoïne, de polyéthylèneglycoléthers ou d'esters de polyéthylèneglycol.

**9.** Cosmétique selon l'une des revendications précédentes, **caractérisé en ce que** la préparation cosmétique se trouve sous forme d'une émulsion.

**10.** Cosmétique selon l'une des revendications précédentes, **caractérisé en ce que** la préparation cosmétique se trouve sous forme d'une émulsion huile-dans-eau (émulsion H/E).

**11.** Cosmétique selon l'une des revendications précédentes, **caractérisé en ce que** la préparation cosmétique contient l'ester de type oxystéarate d'éthylhexyle en une concentration de 0,1 à 10% en poids par rapport au poids total de la préparation.

**12.** Cosmétique selon l'une des revendications précédentes, **caractérisé en ce que** la quantité totale d'ester de type oxystéarate d'éthylhexyle dans la phase huileuse de la préparation représente au moins 3% en poids par rapport au poids total de la préparation.

**13.** Cosmétique selon l'une des revendications précédentes, **caractérisé en ce que** la préparation contient une ou plusieurs huiles choisies dans le groupe des composés octyldodécanol, triglycérides capryliques/capriques (INCI : Caprylic/Capric Triglyceride), glycérides de coco (INCI : Cocoglycerides), stéarate de tridécyle (INCI : Tridecyl Stearate), trimellitate de tridécyle (INCI : Tridecyl Trimellitate).

**14.** Cosmétique selon l'une des revendications précédentes, **caractérisé en ce que** la préparation contient une ou plusieurs huiles choisies dans le groupe des composés contenant polycitronellol (INCI : Polycitronellol), acétate de polycitronellol (INCI : Polycitronellol Acetate), trioléine, diheptanoate de propylèneglycol (INCI : Propylene Glycol Diheptanoate), dicaprate de propylèneglycol (INCI : Propylene Glycol Dicaprate), dicaprylate/dicaprate de propylèneglycol (INCI : Propylene Glycol Dicaprylate/Dicaprate) et dibenzoate de propylèneglycol (INCI : Propylene Glycol Dibenzoate).

**15.** Cosmétique selon l'une des revendications précédentes, **caractérisé en ce que** la préparation contient du tocophérol.

**16.** Cosmétique selon l'une des revendications précédentes, **caractérisé en ce que** la préparation contient une ou plusieurs huiles choisies dans le groupe des composés huile d'amande douce (INCI : Prunus Amygdalus Dulcis Oil) et/ou huile de tournesol (INCI : Helianthus Annuus Seed Oil), huile de ricin hydrogénée (INCI : Hydrogenated Castor Oil).

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CN 102762661 A